(19) Europäisches Patentamt
European Patent Office
Office européen des brevets

(11) Publication number: **0 279 787 A1**

(12) # EUROPEAN PATENT APPLICATION

(21) Application number: 88830033.2

(22) Date of filing: 27.01.88

(51) Int. Cl.[4]: **A 61 K 31/55**
//(A61K31/55,31:415)

(30) Priority: 28.01.87 IT 1918487

(43) Date of publication of application:
24.08.88 Bulletin 88/34

(84) Designated Contracting States:
AT BE CH DE FR GB IT LI LU NL SE

(71) Applicant: ISTITUTO DE ANGELI S.p.A.
Via Serio, 15
I-20139 Milano (IT)

(72) Inventor: Pagani, Ferdinando
Via Marigiola, 18
I-20050 Verano Brianza Milano (IT)

Giachetti, Antonio
Via Guerrini, 3
I-20133 Milano (IT)

Donetti, Arturo
Viale Romagna, 4
I-20133 Milano (IT)

(74) Representative: Aimi, Luciano et al
c/o Società Italiana Brevetti S.p.A. Via Carducci 8
I-20123 Milano (IT)

(54) **Pharmaceutical compositions with synergistic antisecretory action for the healing of gastroduodenal ulcer.**

(57) Pharmaceutical compositions, comprising in combination imidazolylphenylformamidines of general formula:

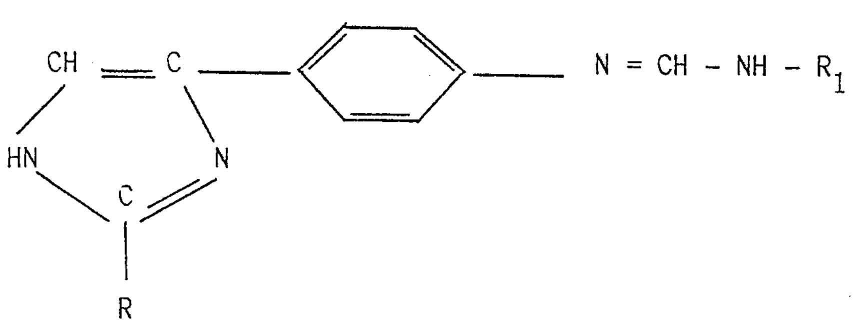

(1)

- in which R represents a hydrogen atom or a methyl group and $R_1$ is an isopropyl group - and pirenzepine, which synergistically inhibit gastric acid secretion and simultaneously reduce the unwanted effects of pirenzepine. These compositions are useful in the treatment of gastroduodenal ulcers and of other diseases of the gastrointestinal tract due to acid hypersecretion.

EP 0 279 787 A1

**Description**

PHARMACEUTICAL COMPOSITIONS WITH SYNERGISTIC ANTISECRETORY ACTION FOR THE HEALING OF GASTRODUODENAL ULCER

The present invention relates to the novel pharmaceutical compositions comprising, in combination, imidazolylphenylformamidines and pirenzepine which synergistically inhibit gastric acid secretion reducing the unwanted effects of pirenzepine in the treatment of gastroduodenal ulcers and of other diseases of the gastrointestinal tract due to acid hypersecretion.

Imidazolylphenylformamidines of general formula:

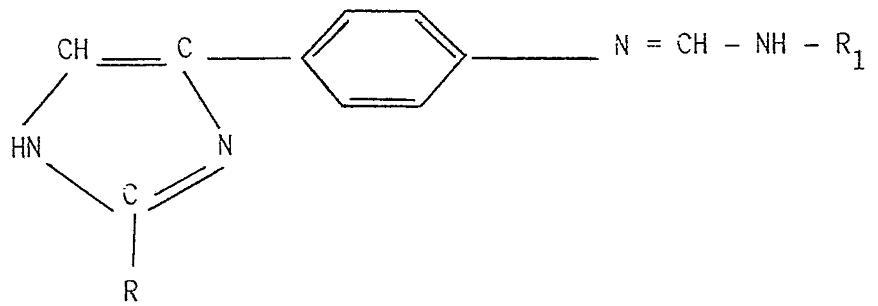

(1)

- in which R represents a hydrogen atom or a methyl group and $R_1$ is an isopropyl group - and physiologically compatible acid-addition salts thereof, are known $H_2$ receptor antagonists which potently inhibit gastric acid secretion. For the purpose of the present invention imidazolylphenylformamidines of formula 1 comprise,when R is a hydrogen atom and $R_1$ is an isopropyl group, mifentidine, (N-isopropyl-N'-[4-(imidazol-4-yl)phenyl]-formamidines), described and claimed in our Italian patent application No. 26323A/80, and, when R is a methyl group and $R_1$ is an isopropyl group, the known DA-5047 (N-isopropyl-N'-[4-(2- -methyl-imidazol-4-yl)phenyl]-formamidine), described and claimed in our Italian patent application No. 22110 A/83.

Pirenzepine is a known selective antagonist of muscarinic $M_1$-receptor. As such it possesses gastric antisecretory activity with a minor incidence of the typical side-effects displayed by classical and non-selective antimuscarinic agents such as dry mouth, mydriasis, constipation, tackicardia, etc. Pirenzepine is currently used in ulcer therapy (G. Bianchi Porro and M. Petrillo, Scand. J.Gastroent. 17, Suppl. 72, 229, 1982). However, clinical studies carried out on pirenzepine have shown that in order to achieve healing rates in a relatively short period pirenzepine must be given in a dose which results in significant side-effects, of which the most common is dry mouth (J. A. Wilson and R. H. Hunt in "Pirenzepine, knowledge and new trends" (1986). Edited by R. Cheli and F. Molinari, Cortina Internazional Verona; Raven Press, New York, pp. 21-27).

The vagal cholinergic pathway is the main determinant of gastric acid secretion. Its action is mediated through the endogenous release of acetylcholine, which in turn participate to the release of the other two segretagogues: histamine and gastrin. In physiological conditions, the full biological effectiveness of the gastric acid secretory process is due to acetylcholine, histamine and gastrin acting in concert on the gastric mucosa. It is known that imidazolylphenylformamidines of formula 1 antagonise the effect of histamine and gastrin, whereas pirenzepine abolishes the muscarinic effect of actylcholine.

We have now found, and this is object of the present invention, that the administration of the pharmaceutical compositions comprising, in a suitable combination, imidazolylphenylformamidines of formula 1 or physiologically compatible acid-addition salts thereof and pirenzepine inhibits the gastric acid secretion at doses lower than wither drug alone, showing a significant synergistic interaction between two drugs and therefore reduces significantly the unwanted effects which may be seen after pirenzepine administration alone.

According to one feature of the present invention we provide pharmaceutical compositions comprising, in combination, imidazolylphenylformamidines for formula 1 or physiologically compatible acid-addition salts thereof and pirenzepine which synergistically inhibit gastric acid secretion reducing the unwanted effects of pirenzepine, in association with pharmaceutical carriers, in the treatment of gastroduodenal ulcers and in other diseases of the gastrointestinal tract due to acid hypersecretion.

The synergistic effect observed when the pharmaceutical compositions comprising in combination, imidazolylphenylformamidines of formula 1 or physiologically compatible acid-addition salt thereof and pirenzepine are administered to animals is shown in the following tables.

Experiments in lumen perfused stomach of the anaesthetized rat were performed, evoking acid secretion by histamin (0.5 mg/kg/h i.v.) and acetylcholine (0.5 mg/kg/h i.v.) given in combination. These two combined secretagogues evoked a response which was about 80% of the maximal effect achievable by maximally effective dose of either acetylcholine (8 mg/kg/h i.v.) or histamine (8 mg/kg/h i.v.) alone.

As soon as a steady level of acid output was reached, a combination of mifentidine/pirenzepine (1:10), or of DA-5047/pirenzepine (1:10) under the form of the compositions of the present invention, or mifentidine,

DA-5047 and pirenzepine alone were administered intravenously. Each dose was administered to 8 animals. A set of six doses of each above drug alone or of the above combinations dependently reduced gastric acid secretion: the acid output before and after administration allowed the calculation of the present inhibition achieved.

In particular at different doses of the combination of mifentidine/pirenzepine, of DA-5047/pirenzepine, and of mifentidine, DA-5047 and pirenzepine administered alone, the percent inhibitions obtained are shown in the following table I:

Table I

| | Dose µg/kg i.v. | Percent inhibition |
|---|---|---|
| Combination of Mifentidine/pirenzepine | 20 : 200 | 51% |
| | 40 : 400 | 75% |
| | 80 : 800 | 89% |
| Combination of DA-5047 /pirenzepine | 20 : 200 | 61% |
| | 40 : 400 | 81% |
| | 80 : 800 | 93% |
| Mifentidine | 20 | 20% |
| | 40 | 36% |
| | 80 | 58% |
| DA-5047 | 20 | 21% |
| | 40 | 34% |
| | 80 | 52% |
| Pirenzepine | 200 | 28% |
| | 400 | 46% |
| | 800 | 62% |

The obtained data were submitted to linear regression analysis in order to calculate the dose inhibiting 50% ($ED_{50}$) the response of which is reported in the following table II:

Table II

| | $ED_{50}$ µ/kg i.v. | | |
|---|---|---|---|
| | Alone Compounds (1.2 and 3) | Combined Compounds (1 and 3) | Combined Compounds (2 and 3) |
| 1 Mifentidine | 71.3 | 16.2 | // |
| 2 DA-5047 | 72.8 | // | 12.8 |
| 3 Pirenzepine | 524.9 | 162 | 128.1 |

From the data reported in Table I it is evident that the combination of mifentidine/pirenzepine and the one of DA-5047/pirenzepine (ratio 1:10) allow a significant and substantial reduction of the respective $ED_{50}$(Table II). These differences are highly significant as demonstrated by statistical analysis for potentiating interaction (T-C. Chou and P. Talalay Eur. J. Bioch. 115, 207-216, 1981). Thus it is apparent that the combinations of imidazolylphenylformamidines of formula 1 or physiologically compatible acid-addition salts thereof with pirenzepine under the form of compositions of the present invention, in particular the combination of mifentidine/pirenzepine and of DA-5047/ pirenzepine are potent synergistic combinations for the reduction of excess gastric acid secretion and thus for the treatment of duodenal and gastric ulcers or other conditions treated until now with the imidazolylphenylformamidines and pirenzepine separately. Furthermore the combination of imidazolylphenylformamidines of formula 1 with pirenzepine under the form of a pharmaceutical composition according to the invention allows reduction of the doses employed in ulcer therapy, with the advantage to reduce significantly the unwanted effects whicy may be seen after pirenzepine administration, enhancing the ulcer healing and antisecretory activity of the imidazolylphenylformamidines of formula 1 and pirenzepine.

The compositions according to the present invention may be administered 1 to 4 times daily, generally as a single dose of from 0.25 mg to 5 mg of imidazolylphenylformamidines of formula 1 or physiolocically compatible acid-addition salts thereof in combination with from 1.25 mg to 100 mg of a pirenzepine.

Within the above ranges, it is preferable that the ratio of imidazolylphenylformamidines or physiologically compatible acid-addition salts thereof to pirenzepine ranges from 1:5 to 1:20, and more preferably 1:10.

For therapeutical use, the compositions, according to the invention, may be made into the usual pharmaceutical forms, such as e.g. tablets, coated tablets, capsules, freeze-dried vials, using conventional galenic excipients such as lactose, corn starch, magnesium stearate, mannitol, polyvinylpyrrolidone.

The following not limitative examples are intended to illustrate the invention:

Example 1

Tablets
- Imidazolylphenylformamidine of formula 1 1.25 mg
- Pirenzepine 12.5 mg
- lactose 253.25 mg
- corn starch 30 mg
- magnesium stearate 3 mg

Method of preparation: the active ingredients, lactose and corn starch were mixed and homogeneously moistened with water. After screening of the moist mass and drying in a tray dryer, the mixture was again passed through a screen and magnesium stearate was added. Then the mixture was pressed into tablets weighing 300 mg each.

Example 2

Capsules
- Imidazolylphenylformamidines of formula 1 1.25 mg
- Pirenzepine 12.5 mg
- corn starch 206.25 mg
- Magnesium stearate 2 mg

Method of preparation: the active ingredients were mixed with the auxiliary products, and the mixture was passed through a screen and mixed homogeneously in a suitable device. The resulting mixture was filled into hard gelatine capsules (222 mg per capsule).

Example 3

Freeze-dried vials
- Imidazolylphenylformamidine of formula 1 0.25 mg
- Pirenzepine 2.5 mg
- Mannitol 9 mg

Method of preparation: the active ingredients were dissolved in suitable amount of water, then the resulting solution was introduced into 5 ml vials sterile conditions and freeze-dried by applying suitable conditions.

**Claims**

1. Pharmaceutical compositions with synergystic effect for the treatment of gastroduodenal ulcers and of other diseases of the gastrointestinal tract due to acid hypersecretion, characterized in that they comprise, in combination, imidazolylphenylformamidines of general formula :

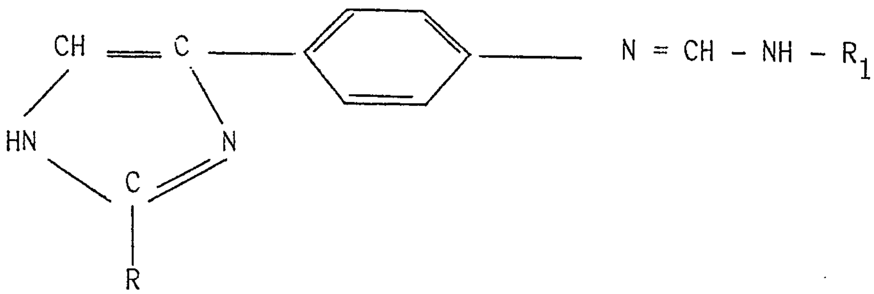

(1)

- where R represents a hydrogen atom or a methyl group and $R_1$ is an isopropyl group - or physiologically compatible acid-addition salts thereof, and pirenzepine as well as pharmaceutical acceptable carriers, being the ratio of two active components included from 1:5 to 1:20.

2. Pharmaceutical compositions as claimed in claim 1 characterized in that the ratio of two active components is 1:10.

3. Pharmaceutical compositions as claimed in claim 1 characterized in that they contain from 0.25 to 5 mg of imidazolylphenylformamidines of formula 1 or physiologically compatible acid-addition salts thereof and from 1.25 to 100 mg of pirenzepine.

4. Pharmaceutical compositions as claimed in claim 1 to 3 characterized in that imidazolylphenylformamidines of formula 1 include mifentidine and DA-5047.

5. Pharmaceutical compositions as claimed in claims 1 to 4 characterized in that they are in the form of tablets, capsules and freeze-dried vials.

European Patent Office

# EUROPEAN SEARCH REPORT

Application Number

EP 88 83 0033

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | UNLISTED DRUGS, vol. 37, no. 4, April 1985, page 68, Chatham, New Jersey, US * Page 68: "Duo vizerul" * --- | 1-5 | A 61 K 31/55 // (A 61 K 31/55 A 61 K 31:415) |
| A | EP-A-0 053 407 (ISTITUTO DE ANGELI S.p.A.) ----- | 1-5 | |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 16-05-1988 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)